# EUROPÄISCHE PATENTSCHRIFT

(11) **EP 0 985 733 B1**
(45) Veröffentlichungstag und Bekanntmachung des Hinweises auf die Patenterteilung: **04.03.2009**
(21) Anmeldenummer: 99890178.9
(22) Anmeldetag: 02.06.1999
(51) Int. Cl.: C12P 19/18, C08B 37/16

(54) **Verfahren zur Herstellung von Cyclodextrin**
Process for the preparation of cyclodextrin
Procédé pour la préparation de cyclodextrine

(30) Priorität: 11.08.1998 AT 138098
(43) Veröffentlichungstag der Anmeldung: 15.03.2000
(73) Patentinhaber: Südzucker Aktiengesellschaft Mannheim/Ochsenfurt, 68165 Mannheim (DE)
(72) Erfinder: Grüll, Dietmar, Dr., 3442 Langenschönbichl (AT); Stifter, Ulrich , Dr., 3400 Klosterneuburg (AT)
(74) Vertreter: Pawloy, Peter Michael

(56) Entgegenhaltungen:
- WO-A-92/11376
- WO-A-93/10255
- US-A- 3 425 910
- US-A- 4 477 568

## Beschreibung

Gegenstand der vorliegenden Erfindung ist ein Verfahren zur Herstellung von Cyclodextrin aus Amylopektin-Kartoffelstärke durch Einwirkung von Cyclodextrin-Glycosyltransferase (CGTase, auch Cyclodextrin-Transglycosylase genannt, EC Nr. 2.4.1.19).

Unter Cyclodextrinen versteht man eine Gruppe von Substanzen, deren Moleküle aus mehreren Anhydroglucoseeinheiten mit zyklisch verknüpften alpha-1,4-glucosidischen Bindungen bestehen, wobei das kleinste Cyclodextrin, alpha-Cyclodextrin, 6 Glucoseeinheiten enthält. Nach der Anzahl der im entstehenden Ring enthaltenen Anhydroglucoseeinheiten unterscheidet man außerdem noch beta- und gamma-Cyclodextrin. beta-Cyclodextrin ist das thermodynamisch begünstigte Reaktionsprodukt bei der Umwandlung von Stärke in Cyclodextrin mit Hilfe von CGTase. Bevorzugt für technische Verwendungen sind die höherringigen Cyclodextrine.

In technischen Verfahren zur Herstellung von Cyclodextrinen wurden bisher folgende Stärken als hauptsächliche Ausgangssubstanzen eingesetzt:

Kartoffelstärke: die Kartoffel kann auch in schlechten Lagen mit hohen Hektarerträgen angebaut werden. Sie hat einen geringen Protein- und Lipidgehalt und liefert daher eine sehr reine Stärke.

Mais- und Wachsmaisstärke: Mais benötigt ein wärmeres Klima. Wachsmais hat hohe Reifezahlen. Der Anbau bleibt auf Gunstlagen mit ausreichender Abgrenzung von normalem Mais beschränkt. Geringe Hektarerträge verursachen eine weitere Verteuerung.

Sehr nachteilig bei Stärke aus Mais und Wachsmais ist der hohe Protein- und Lipidgehalt (es ist eine aufwendige und teure Reinigung der Stärke notwendig).

Weizenstärke: stellt ein schlechteres Substrat als Kartoffel- und Maisstärke dar, da sie eine deutlich geringere Ausbeute bei der Cyclodextrinherstellung ergibt.

Die üblichen natürlichen Stärken stellen ein Gemisch aus den beiden Stärkeformen Amylopektin und Amylose dar. Amylose und Amylopektin sind ihrerseits wieder keine einheitlichen Substanzen, sondern sind Gemische von Polymeren mit unterschiedlichen Molekulargewichten und unterschiedlichen Glucose-Bindungen. Amylose besteht im wesentlichen aus unverzweigten Polysacchariden, in denen die Glucose in alpha-1,4-Bindung vorliegt. Amylopektin hingegen ist ein stark verzweigtes Glucosepolymer, bei dem die Glucoseeinheiten neben den alpha-1,4-Bindungen an den Verzweigungsstellen in 1,6-Bindung enthalten sind. Es hat sich gezeigt, daß Amylopektin stabilere Lösungen als Amylose ergibt, da Amylose zu unerwünschter Retrogradation, d.h. einem Wiederzusammenschluß bereits voneinander getrennter Ketten, neigt.

Die üblichen natürlichen Stärken enthalten unabhängig von der Pflanzenart, aus der sie gewonnen wurden, 15 % bis 30 % Amylose. Nur Maissorten des sogenannten Waxy-Typs liefern eine Stärke, die fast ausschließlich aus Amylopektin besteht. In seltenen Fällen kann eine amylopektinreiche Stärke auch aus sogenanntem Wachsreis oder aus Wachsgerste gewonnen werden.

Amylose und Amylopektin können durch Fraktionierung voneinander getrennt werden. Diese Verfahren sind sehr aufwendig und kostenintensiv und werden außer im Laboratoriumsmaßstab kaum durchgeführt. Außerdem wird bei der Fraktionierung der natürlichen Stärke ein unkontrollierter Abbau und eine Schädigung der Stärkefraktionen hervorgerufen, wodurch die Eigenschaften der derart gewonnenen Produkte nachteilig beeinflußt werden.

Für technische Zwecke wird daher Amylopektin-Stärke praktisch kaum eingesetzt. In der Praxis findet nur die natürlich vorkommende Wachsmaisstärke im Lebensmittelbereich eine gewisse Anwendung, da sie ein angenehmeres Mundgefühl als gewöhnliche Stärke bewirkt.

Die Herstellung von Cyclodextrin aus Stärke ist in zahlreichen Literaturstellen behandelt.

So beschreibt die US-PS 3,425.910 ein Verfahren zur Herstellung von Cyclodextrin aus einem Stärkehydrolysat. Die Verwendung von Kartoffelstärke als Stärke-Ausgangsmaterial wird erwähnt. Zum Zeitpunkt der Hinterlegung des genannten US-Patentes (1966) ist die Kartoffelstärke eine übliche Stärke mit einem Amylosegehalt von etwa 20 Gew.-%.

In der PCT-Anmeldung WO 93/10255 wird die Herstellung von Cyclodextrin aus einer mindestens 90 % Amylopektin enthaltenden Stärke beschrieben, wodurch ein Cyclodextrin erhalten werden soll, welches eine trübungsfreie Lösung in Wasser ergibt. Bevorzugte Ausgangsstärken enthalten 95 % oder mehr, insbesondere etwa 99 %, Amylopektin. Es werden konkret Wachsmaisstärke, Wachsreisstärke und Wachsgerstenstärke als Ausgangsmaterial angegeben, wobei Wachsmaisstärke als bevorzugt genannt ist. Die Vergleichsstärken, die im Beispiel 1 zur Demonstration des positiven Effekts der Verwendung von Wachsmaisstärke angegeben sind, sind Kartoffelstärke und Maisstärke mit den üblichen Amylosegehalten.

Zu den in der genannten PCT-Anmeldung ausgeführten Verfahrensbedingungen zählt die Verwendung eines Komplexierungsmittels für das Cylcodextrin mit dem Zweck der besseren Abscheidung desselben aus dem Reaktionsmedium. Als Komplexierungsmittel sind Toluol, 1-Decanol, Cyclodecanol, Cyclohexan, Trichlorethylen, Tetrachlorethan, Brombenzol, 2,3-Cyclododenopyridin, Naphthalin, 1-Naphthol, 2-Naphthol und Dimethylphenol genannt.

In J. Szejtli und T. Ose, Comprehensive Supramolecular Chemistry, Bd. 3, Cyclodextrins, 1996, Pergamon, Oxford, UK, wird in Artikel 3 auf Seite 41, Preparation and Industrial Production of Cyclodextrins, G. Schmid, Wacker-Chemie GmbH, München, Deutschland, ein Überblick über die industrielle Herstellung von Cyclodextrinen gegeben.

Es werden die verschiedenen Umwandlungsbedingungen und ihre Auswirkung auf das Verhältnis von alpha-, beta- und gamma-Cyclodextrin beschrieben. Dabei kann der Zusatz eines speziellen Komplexierungsmittels während des Herstellungsverfahrens auch das Verhältnis der drei Arten des Cyclodextrins zueinander verändern.

Weiters ist erwähnt, daß die alpha-1,6-glucosidischen Bindungen an den Verzweigungspunkten im Amylopektin die Wirkung der CGTase blockieren. Wenn man Entzweigungsenzyme, wie z.B. Pullulanase oder Isoamylase, vor dem Zusatz von CGTase auf das Amylopektin einwirken läßt, so erhöht sich der Umwandlungsgrad der Stärke in Cyclodextrin um etliche Prozente.

In der Folge wird in der genannten Literaturstelle Szejtli und Ose auch erwähnt, daß Amylopektin ein besseres Substrat für die Cyclodextrin-Herstellung ist als Amylose, da die CGTase ausgehend von den nicht-reduzierenden Enden des Stärkemoleküls auf dasselbe einwirkt. Da Amylopektin wesentlich mehr nicht-reduzierende Enden als Amylose aufweist, ist der Umsetzungsgrad bei Verwendung von Amylopektin besser. Es wird daher empfohlen, Kartoffelstärke statt Maisstärke zu verwenden, da Kartoffelstärke von Natur aus einen etwas höheren Amylopektingehalt hat als Maisstärke (etwa 79 % bei Kartoffel im Vergleich zu etwa 72 % bei Mais).

Schließlich wird noch der Versuch beschrieben, das Cyclodextrin direkt in den Knollen von transgenen Kartoffelpflanzen durch Konstruktion eines chimärischen Gens mit Hilfe des CGTase-Gens von Klebsiella oxytoca herzustellen. Tatsächlich konnten dabei geringe Mengen Cyclodextrin in den Kartoffelknollen nachgewiesen werden. Die Extraktion des Knollengewebes erfolgte durch eine C18 Sep-pak-Säule, die das Cyclodextrin, jedoch nicht die Stärke bindet.

In der US-PS 4 477 568 wird für die Herstellung von Cyclodextrin u.a. die Verwendung von fraktionierter Amylopektinstärke aus den verschiedensten Erntefrüchten, wie Mais, Weizen, Sorghum, Kartoffeln, Tapioka, Sago und Reis erwähnt.

Da sich jedoch die Stärkefraktionierverfahren aus den früher erwähnten Gründen nicht durchgesetzt haben, ist man nach wie vor auf der Suche nach einem Ausgangsmaterial für die Cyclodextrinherstellung, bei dem die genannten Nachteile vermieden werden.

Gemäß der vorliegenden Erfindung wird bei einem Verfahren zur Herstellung von Cyclodextrin aus Amylopektin-Kartoffelstärke durch Einwirkung von Cyclodextrin-Glycosyltranslerase als Stärke-Ausgangsmaterial eine Amylopektin-Kartoffelstärke eingesetzt, die aus durch Züchtung oder molekularbiologische bzw. gentechnische Verfahren hinsichtlich der Amylosebildung inhibierten Kartoffeln gewonnen wird.

In den letzten Jahren wurde die gentechnische Veränderung von Kartoffeln mit dem Zweck der Produktion von praktisch amylosefreier Stärke erfolgreich entwickelt. Die aus solchen Kartoffeln gewonnene Amylopektin-Kartoffelstärke kombiniert die Vorteile eines praktisch reinen Amylopektins, das die ursprünglichen Eigenschaften des Natur-produktes aufweist, mit den Vorteilen der Kartoffelstärke, nämlich dem geringen Lipid- und Proteingehalt.

Am besten wird die Amylopektin-Kartoffelstärke aus durch anti-sense-Technik oder durch Cosuppression hinsichtlich der Amylosebildung inhibierten Kartoffeln gewonnen.

Die WO 92/11376 beschreibt die gentechnische Modifikation der Kartoffel durch antisense-Technik im Hinblick auf die Inhibierung der Amylosebildung.

Die Kartoffelsorten, die für den vorliegenden Fall als Produzenten der Amylopektin-Starke eingesetzt werden, liefern eine Amylopektin-Stärke mit einem Amylopektingehalt von über 90 Gew.-%, vorzugsweise über 95 %. Besonders bevorzugt wird für das erfindungsgemäße Verfahren eine Amylopektin-Kartoffelstärke mit einem Amylopektingehalt von über 98 % verwendet.

Die Ermittlung des Amylosegehalts bzw. des Amylopekingehalts einer Stärke erfolgt nach: J.H.M. Hovenkamp-Hermelink, J.N. DeVries, F. Adamse, E. Jacobsen, W. Witholt und W.J. Feenstra, "Rapid estimation of the amylose amylopectin ratio in small amounts of tuber and leave tissue of the potatoe", Potatoe Res. 31, [1988], 241-246.

Das Enzym Cyclodextrin-Glycosyltransferase (EC Nr. 2.4.1.19], wird durch Züchtung von Mikroorganismen, die dieses Enzym produzieren, und Gewinnung des Enzyms aus der Fermentationsbrühe hergestellt. B. macerans ist ein Beispiel eines solchen Mikroorganismus.

Die Cyclodextrine bildern auf Grund des in ihren Inneren vorliegenden Hohlraums Einschlußverbindungen oder Komplexe mit verschiedenen kleineren Molekülen oder Monomeren. Es ist anzunehmen, daß diese Komplexbildung über die hydrophobe Wechselwirkung zwischen dem apolaren Ringinneren der Cyclodextrine und den ebenfalls apolaren Gasmolekülen sowie mit Hilfe von van-der-Waal'sChen Kräften erfolgt. Die chemischen und physikalischen Eigenschaften der Gastmoleküle werden durch den Einschluß in die Cyclodextrine so verändert, daß dabei gezielte Modifikationen der Gastmoleküle erreicht werden können.

Zu den vorteilhaften Wirkungen der Komplexierung mit Cyclodextrin zählen folgende:
1. Stabilisierung licht- oder sauerstoffempfindlicher Substanzen.
2. Änderung der chemischen Reaktivität von Gastmolekülen:
   a) Reaktive Substanzen werden durch Einschluß geschützt und können gefahrlos mit anderen Substanzen gemischt werden;
   b) Reaktionen können durch Einschluß funktioneller Gruppen in ihrer Selektivität beeinflußt werden;
   c) Reaktionen können gefördert oder unterdrückt werden.
3. Fixierung leichtflüchtiger Substanzen:
   a) Lagerung und Handhabung werden erleichtert, besonders bei toxischen Substanzen;
   b) Die notwendige Menge an flüchtigen Substanzen kann verringert werden, da der Verdunstungsverlust herabgesetzt wird;
   c) Aromastoffe und physiologisch wirksame Substanzen lassen sich besser dosieren.
4. Änderung anwendungsbezogener Eigenschaften der Gastmoleküle:
   a) In Wasser schlecht lösliche Substanzen werden bei Zugabe von Cyclodextrinen besser löslich und können leichter emulgiert werden;
   b) Pulverisierte, gefriergetrocknete Cyclodextrinkomplexe liegen feindispers vor und sind leichter löslich als die in Wasser schwer löslichen unkomplexierten Gastmoleküle;
   c) Pigmente können maskiert oder Farbtöne von Substanzen geändert werden, da sich bei Einschluß im allgemeinen das Absorptionsspektrum ändert;
   d) Unangenehme Geschmackstoffe können unterdrückt werden.

Besonders im Lebensmittelbereich wird zukünftig ein vermehrter Einsatz von Cyclodextrinen erwartet, wenn es gelingt, seine Produktionskosten zu senken.

Die erfindungsgemäß eingesetzte Amylopektin-Kartoffelstärke kann unbehandelt oder mechanisch, thermisch, chemisch und/oder enzymatisch vorbehandelt sein. Diese Vorbehandlung dient einer Verfüssigung bzw. besseren Löslichmachung der Stärke.

Bei der mechanischen Vorbehandlung wird die Amylopektin-Kartoffelstärke durch hochtouriges Rühren verflüssigt.

Die Stärke kann auch thermisch bei Temperaturen bis ca 155°C vorbehandelt werden.

Unter chemischer Vorbehandlung versteht man in der Regel eine Behandlung mit Säure, vorzugsweise mit Salzsäure.

Andererseits kann die Stärke aber auch mit Oxidationsmitteln, wie z.B. Natriumhypochlorit, vorbehandelt werden.

Wird eine Amylopektin-Kartoffelstärke mit alpha-Amylase behandelt, so erfolgt dabei ein enzymatischer Abbau, der die Stärke ebenfalls leichter löslich macht.

Eine chemische Vorbehandlung zur Herstellung von Stärkeethern, -estern und/oder vernetzten Stärkeprodukten wird ebenfalls mit Vorteil eingesetzt.

Es hat sich auch im vorliegenden Fall herausgestellt, daß eine Behandlung mit einem Entzweigungsenzym, wie Pullulanase (EC 3.2.1.41) oder Isoamylase (EC 3.2.1.68), einen guten Einfluß auf die Ausbeute des Cyclodextrins hat.

Ebenso wirkt sich auch beim erfindungsgemäßen Verfahren die Verwendung eines Komplexierungsmittels günstig aus.

Die folgende Tabelle 1 zeigt die Steigerung der Ausbeute bei Verwendung von Pullulanase bzw. bei gemeinsamer Verwendung von Pullulanase und einem Komplexierungsmittel.

Die Stärkesuspension wird bei 100°C vorbehandelt, die Cyclisierungsreaktion erfolgt dann bei 25°C.

**Tabelle 1**

| Eingesetztes Substrat | Ausbeute an CD % | Ausbeute bei Verwendung v. Pullulanase % | Ausbeute bei Verwendung v. Pullulanase und Komplex.-mittel % |
|---|---|---|---|
| frakt. Mais-AP | 22,6 | 36,1 | 89,8 |
| Maisstärke | 14 | | 87,2 |
| Wachsmaisstärke | 18,6 | | 90,6 |
| Kartoffelstärke | 18,9 | | 85,9 |
| Kartoffel-AP | | | |
| aus transgener Kart. | 25,1 | 38,3 | 92,3 |
| Weizenstärke | 15,8 | | 86,9 |

Für die höchsten Ausbeuten an Cyclodextrinen bei Verwendung von amylosefreier Kartoffelstärke wird folgende Erklärung vermutet:

Bei Verwendung von Isoamylase oder Pullulanase als Entzweigungsenzym entstehen Bruchstücke mit Kettenlängen von DP 60 und 18. Die folgende Tabelle 2 zeigt die DP-Verteilung verschiedener Amylopektine, die mit Isoamylase entzweigt sind.

**Tabelle 2**

| Amylopektin | DP Fraktion 1 | DP Fraktion 2 | Gew.Verh.F1:F2 |
|---|---|---|---|
| Kartoffel | 60 | 18 | 1:2,1 |
| Mais | 45 | 15 | 1:3,5 |
| Weizen | 49 | 13 | 1:4,8 |

Der Anteil der kleinen Bruchstücke ist bei der Kartoffelstärke am niedrigsten (M.T. Kalichevsky, P.D. Orford und S.G. Ring, "The retrogradation and gelation of amylopectins from various botanical sources", Carbohyd. Res., 198 (1990) 49-55). Die höchsten Ausbeuten an Cyclodextrinen werden mit Stärken von DE ≤2 erhalten (F.C. Armbruster und E.R. Kooi, Production of Cyclodextrin, US-Patent 3,425.910).

Die Ausbeuten an Cyclodextrinen bei Verwendung von Amylopektin-Stärke aus transgener Kartoffel sind höher als die Ausbeuten, die bei Ansätzen mit Wachsmaisstärke erhalten werden (J.W. Shieh und A. Hedges, PCT-Anmeldung WO 93/10255 (1993)). Als Erklärung wird der höhere Dextrinanteil der Fraktion 1 mit DP 60 vermutet.

Es hat sich gezeigt, daß mit besonderem Vorteil eine Amylpektin-Kartoffelstärke mit einem Polymerisationsgrad (DP) von ≥ 50 eingesetzt wird. Wie aus der folgenden Tabelle 3 ersichtlich ist, steigt die Ausbeute an Cyclodextrin bei Verwendung einer Ausgangsstärke mit steigendem DP.

**Tabelle 3**

| % Ausbeute Cyclodextrin | DP |
|---|---|
| 51,2 | 10 |
| 70,7 | 20 |
| 92,3 | ≥ 50 |

Vorteilhaft bei der Isolierung der Cyclodextrine aus dem Reaktionsansatz ist die hohe Reinheit der Kartoffelstärke (geringer Fett- und Proteingehalt), die sich beispielsweise in einer Verbesserung der Transmission manifestiert. In der folgenden Tabelle 4 ist der Protein- und Lipidgehalt kommerzieller Stärken angegeben:

**Tabelle 4**

| Stärke | Protein | Lipid |
|---|---|---|
| | % i.TS | |
| Mais | 0,2-0,4 | 0,5-0,9 |
| Kartoffel | 0,05-0,1 | 0-0,1 |

### Beispiel

100 g amylosefreie Kartoffelstärke aus transgener Kartoffel werden in 1 Liter Wasser suspendiert und durch Erhitzen auf 100°C innerhalb von 30 Minuten zum Gelieren gebracht. Nach dem Abkühlen auf 25°C werden entweder 16,5 ml Pullulanase-Suspension (45 U/ml) oder 0,5 ml Isoamylase-Suspension (5330000 U/ml) zugegeben. Der Ansatz wird 3 Stunden gerührt. Dann werden 10 mg Cyclodextrin-Glycosyltransferase und Cyclodecanon als Komplexierungsmittel zugesetzt. Diese Reaktionsmischung wird 10 Tage gerührt. Die Ausbeute an Cyclodextrinen beträgt bei Verwendung von Pullulanase 92,3 %.

## Patentansprüche

1. Verfahren zur Herstellung von Cyclodextrin aus Amylopektin-Kartoffelstärke durch Einwirkung von Cyclodextrin-Glycosyltransferase (EC Nr. 2.4.1.19), **dadurch gekennzeichnet, dass** als Stärke-Ausgangsmaterial eine Amylopektin-Kartoffelstärke eingesetzt wird, die aus durch Züchtung oder gentechnische bzw. andere molekular-biologische Verfahren hinsichtlich der Amylosebildung inhibierten Kartoffeln gewonnen wurden.

2. Verfahren nach Anspruch 1, **dadurch gekennzeichnet, dass** eine Amylopeltin-Kartoffelstärke eingesetzt wird, die aus durch antisense-Technik hinsichtlich der Amylosebildung inhibierten Kartoffeln gewonnen wurde.

3. Verfahren nach Anspruch 1, **dadurch gekennzeichnet, dass** eine Amylopektin-Kartoffelstärke eingesetzt wird, die aus durch Co-suppression hinsichtlich der Amylosebildung inhibierten Kartoffeln gewonnen wurde.

4. Verfahren nach einem der Ansprüche 1 bis 3, **dadurch gekennzeichnet, dass** eine Amylopektin-Kartoffelstärke mit einem Amylopektingehalt von mindestens 95% eingesetzt wird.

5. Verfahren nach einem der Ansprüche 1 bis 3, **dadurch gekennzeichnet, dass** eine Amylopektin-Kartoffelstärke mit einem Amylopektingehalt von mindestens 98% eingesetzt wird.

6. Verfahren nach einem der Ansprüche 1 bis 5, **dadurch gekennzeichnet, dass** eine mechanisch und/oder thermisch und/oder chemisch und/oder enzymatisch vorbehandelte Amylopektin-Kartoffelstärke eingesetzt wird.

7. Verfahren nach Anspruch 6, **dadurch gekennzeichnet, dass** eine mechanisch durch hochtouriges Rühren verflüssigte Amylopektin-Kartoffelstärke eingesetzt wird.

8. Verfahren nach Anspruch 6, **dadurch gekennzeichnet, dass** eine thermisch bei Temperaturen bis ca. 155°C behandelte Amylopektin-Kartoffelstärke eingesetzt wird.

9. Verfahren nach Anspruch 6, **dadurch gekennzeichnet, dass** eine mit Säure behandelte Amylopektin-Kartoffelstärke eingesetzt wird.

10. Verfahren nach Anspruch 6, **dadurch gekennzeichnet, dass** eine mit Salzsäure behandelte Amylopektin-Kartoffelstärke eingesetzt wird.

11. Verfahren nach Anspruch 6, **dadurch gekennzeichnet, dass** eine mit einem Oxidationsmittel, vorzugsweise mit Natriumhypochlorit, behandelte Amylopektin-Kartoffelstärke eingesetzt wird.

12. Verfahren nach Anspruch 6, **dadurch gekennzeichnet, dass** eine mit Natriumhypochlorit behandelte Amylopektin-Kartoffelstärke eingesetzt wird.

13. Verfahren nach Anspruch 6, **dadurch gekennzeichnet, dass** eine mit alpha-Amylase behandelte Amylopektin-Kartoffelstärke eingesetzt wird.

14. Verfahren nach Anspruch 6, **dadurch gekennzeichnet, dass** eine veretherte, veresterte und/oder vernetzte Amylopektin-Kartoffelstärke eingesetzt wird.

15. Verfahren nach Anspruch 6, **dadurch gekennzeichnet, dass** eine mit einem Entzweigungsenzym behandelte Amylopektin-Kartoffelstärke eingesetzt wird.

16. Verfahren nach Anspruch 15, **dadurch gekennzeichnet, dass** eine mit Isoamylase (EC 3.2.1.68) oder Pullulanase (EC 3.2.1.41) behandelte Amylopektin-Kartoffelstärke verwendet wird.

17. Verfahren nach einem der Ansprüche 6 bis 16, **dadurch gekennzeichnet, dass** eine Amylopektin-Kartoffelstärke mit einem durchschnittlichen Polymerisationsgrad ≥ 50 verwendet wird.

18. Verfahren nach einem der Ansprüche 1 bis 17, **dadurch gekennzeichnet, dass** die Umwandlung der Stärke mit Cyclodextrin-Glycosyltransferase in Gegenwart eines Komplexierungsmittels für das Cyclodextrin vorgenommen wird.

19. Verwendung von einer aus durch Züchtung oder gentechnische bzw. andere molekular-biologische Verfahren hinsichtlich der Amylosebildung inhibierten Kartoffeln gewonnene Amylopektin-Kartoffelstärke als Stärke-Ausgangsmaterial für die Herstellung von Cyclodextrin durch Cyclodextrin-Glycosyltransferase-Behandlung von Stärke nach einem der in den Ansprüchen 1 bis 18 genannten Verfahren.

## Claims

1. A process for the production of cyclodextrin from amylopectin potato starch by reaction with cyclodextrin glycosyltransferase (EC No. 2.4.1.19), **characterized in that** an amylopectin potato starch is used as a starting material, which starch has been obtained from potatoes having amylose formation inhibited as a result of breeding or genetic engineering and/or other molecular biological procedures.

2. The process according to claim 1, **characterized in that** an amylopectin potato starch is used which has been obtained from potatoes having amylose formation inhibited as a result of antisense technique.

3. The process according to claim 1, **characterized in that** an amylopectin potato starch is used which has been obtained from potatoes having amylose formation inhibited as a result of cosuppression.

4. The process according to any one of claims 1 to 3, **characterized in that** an amylopectin potato starch is used which has an amylopectin content of at least 95%.

5. The process according to any one of claims 1 to 3, **characterized in that** an amylopectin potato starch is used which has an amylopectin content of at least 98%.

6. The process according to any one of claims 1 to 5, **characterized in that** an amylopectin potato starch is used which has been pretreated mechanically and/or thermally and/or chemically and/or enzymatically.

7. The process according to claim 6, **characterized in that** an amylopectin potato starch is used which has been mechanically liquified by high-speed stirring.

8. The process according to claim 6, **characterized in that** an amylopectin potato starch is used which has been thermally treated at temperatures of up to about 155°C.

9. The process according to claim 6, **characterized in that** an acid-treated amylopectin potato starch is used.

10. The process according to claim 6, **characterized in that** a hydrochloric-acid-treated amylopectin potato starch is used.

11. The process according to claim 6, **characterized in that** an amylopectin potato starch is used which has been treated with an oxidizing agent, preferably with sodium hypochlorite.

12. The process according to claim 6, **characterized in that** a sodium-hypochlorite-treated amylopectin potato starch is used.

13. The process according to claim 6, **characterized in that** an alpha-amylase-treated amylopectin potato starch is used.

14. The process according to claim 6, **characterized in that** an etherified, esterified and/or cross-linked amylopectin potato starch is used.

15. The process according to claim 6, **characterized in that** an amylopectin potato starch is used which has been treated with a debranching enzyme.

16. The process according to claim 15, **characterized in that** an amylopectin potato starch is used which has been treated with isoamylase (EC 3.2.1.68) or pullulanase (EC 3.2.1.41).

17. The process according to any one of claims 6 to 16, **characterized in that** an amylopectin potato starch is used which has an average degree of polymerization of ≥ 50.

18. The process according to any one of claims 1 to 17, **characterized in that** the conversion of the starch with cyclodextrin glycosyltransferase is effected in the presence of a complexing agent for cyclodextrin.

19. The use of an amylopectin potato starch as a starting material for the production of cyclodextrin by cyclodextrin-glycosyltransferase treatment of starch according to a method as defined in claims 1 to 18, with said starch being obtained from potatoes having amylose formation inhibited as a result of breeding or genetic engineering and/or other molecular biological procedures.

## Revendications

1. Procédé de production de cyclodextrine à partir d'amidon de pommes de terre de type amylopectine par action de cyclodextrine-glycosyltransférase (EC N° 2.4.1.19), **caractérisé en ce qu'**un amidon de pommes de terre de type amylopectine qui a été obtenu à partir de pommes de terre inhibées en ce qui concerne la formation d'amylose par culture ou par des procédés de génie génétique ou d'autres procédés de biologie moléculaire est utilisé comme matière première d'amidon.

2. Procédé selon la revendication 1, **caractérisé en ce qu'**un amidon de pommes de terre de type amylopectine qui a été obtenu à partir de pommes de terre inhibées en ce qui concerne la formation d'amylose par la technique antisens est utilisé.

3. Procédé selon la revendication 1, **caractérisé en ce qu'**un amidon de pommes de terre de type amylopectine qui a été obtenu à partir de pommes de terre inhibées en ce qui concerne la formation d'amylose par co-suppression est utilisé.

4. Procédé selon l'une des revendications 1 à 3, **caractérisé en ce qu'**un amidon de pommes de terre de type amylopectine ayant une teneur en amylopectine d'au moins 95 % est utilisé.

5. Procédé selon l'une des revendications 1 à 3, **caractérisé en ce qu'**un amidon de pommes de terre de type amylopectine ayant une teneur en amylopectine d'au moins 98 % est utilisé.

6. Procédé selon l'une des revendications 1 à 5, **caractérisé en ce qu'**un amidon de pommes de terre de type amylopectine prétraité mécaniquement et/ou thermiquement et/ou chimiquement et/ou enzymatiquement est utilisé.

7. Procédé selon la revendication 6, **caractérisé en ce qu'**un amidon de pommes de terre de type amylopectine liquéfié mécaniquement par agitation à grande vitesse de rotation est utilisé.

8. Procédé selon la revendication 6, **caractérisé en ce qu'**un amidon de pommes de terre de type amylopectine traité thermiquement à des températures pouvant atteindre environ 155°C est utilisé.

9. Procédé selon la revendication 6, **caractérisé en ce qu'**un amidon de pommes de terre de type amylopectine traité avec un acide est utilisé.

10. Procédé selon la revendication 6, **caractérisé en ce qu'**un amidon de pommes de terre de type amylopectine traité avec l'acide chlorhydrique est utilisé.

11. Procédé selon la revendication 6, **caractérisé en ce qu'**un amidon de pommes de terre de type amylopectine traité avec un oxydant, de préférence avec l'hypochlorite de sodium, est utilisé.

12. Procédé selon la revendication 6, **caractérisé en ce qu'**un amidon de pommes de terre de type amylopectine traité avec l'hypochlorite de sodium est utilisé.

13. Procédé selon la revendication 6, **caractérisé en ce qu'**un amidon de pommes de terre de type amylopectine traité avec l'alpha-amylase est utilisé.

14. Procédé selon la revendication 6, **caractérisé en ce qu'**un amidon de pommes de terre de type amylopectine éthérifié, estérifié et/ou réticulé est utilisé.

15. Procédé selon la revendication 6, **caractérisé en ce qu'**un amidon de pommes de terre de type amylopectine traité avec une enzyme déramifiante est utilisé.

16. Procédé selon la revendication 15, **caractérisé en ce qu'**un amidon de pommes de terre de type amylopectine traité avec l'isoamylase (EC 3.2.1.68) ou la pullulanase (EC 3.2.1.41) est utilisé.

17. Procédé selon l'une des revendications 6 à 16, **caractérisé en ce qu'**un amidon de pommes de terre de type amylopectine ayant un degré moyen de polymérisation ≥ 50 est utilisé.

18. Procédé selon l'une des revendications 1 à 17, **caractérisé en ce que** la conversion de l'amidon avec la cyclodextrine-glycosyltransférase est réalisée en présence d'un complexant pour la cyclodextrine.

19. Utilisation d'un amidon de pommes de terre de type amylopectine obtenu à partir de pommes de terre inhibées en ce qui concerne la formation d'amylose par culture ou par des procédés de génie génétique ou d'autres procédés de biologie moléculaire comme matière première d'amidon pour la production de cyclodextrine par traitement d'amidon avec la cyclodextrine-glycosyltransférase selon l'un des procédés cités dans les revendications 1 à 18.
